Europäisches Patentamt

European Patent Office

Office européen des brevets

(19)

(11) Veröffentlichungsnummer: **0 188 770 B1**

# EUROPÄISCHE PATENTSCHRIFT

(12)

(45) Veröffentlichungstag der Patentschrift: **15.05.91**

(21) Anmeldenummer: **85116214.9**

(22) Anmeldetag: **19.12.85**

(51) Int. Cl.⁵: **C12N 9/18**, //(C12N9/18, C12R1:38)

Die Akte enthält technische Angaben, die nach dem Eingang der Anmeldung eingereicht wurden und die nicht in dieser Patentschrift enthalten sind.

(54) Verfahren zur Gewinnung von Cholesterinesterase.

(30) Priorität: **24.12.84 DE 3447390**

(43) Veröffentlichungstag der Anmeldung:
**30.07.86 Patentblatt 86/31**

(45) Bekanntmachung des Hinweises auf die Patenterteilung:
**15.05.91 Patentblatt 91/20**

(84) Benannte Vertragsstaaten:
**AT BE CH DE FR GB IT LI LU NL SE**

(56) Entgegenhaltungen:
**EP-A- 0 024 345**
**FR-A- 2 029 678**
**US-A- 3 772 152**
**US-A- 4 011 138**

(73) Patentinhaber: **BOEHRINGER MANNHEIM GMBH**
**Patentabteilung, Abt. E Sandhofer Strasse 112-132 Postfach 31 01 20**
**W-6800 Mannheim 31 Waldhof(DE)**

(72) Erfinder: **Wania, Wolfgang**
**Königbergstrasse 5**
**W-8121 Sindelsdorf(DE)**
Erfinder: **Wahl, Jürgen, Dr.**
**Mittelstrasse 31**
**W-8118 Schlehdorf(DE)**
Erfinder: **Kellner, Maximilian, Dr.**
**Auweg 29**
**W-8110 Seehausen(DE)**

(74) Vertreter: **Weickmann, Heinrich, Dipl.-Ing. et al**
**Patentanwälte Dipl.-Ing. H.Weickmann Dipl.-Phys.Dr. K.Fincke Dipl.-Ing.**
**F.A.Weickmann Dipl.-Chem. B. Huber Dr.-Ing. H. Liska Dipl.-Phys.Dr. J. Prechtel Postfach 860820**
**W-8000 München 86(DE)**

## Beschreibung

Die Erfindung betrifft ein Verfahren zur Gewinnung von Cholesterinesterase aus Mikroorganismen. Cholesterinesterase spielt eine wichtige Rolle in der klinischen Analytik, seit Verfahren zur enzymatischen Bestimmung von Cholesterin entwickelt wurden.

Da ein großer Teil des Cholesterins in biologischem Material in Form von Estern vorliegt, ermöglicht die gemeinsame Verwendung von Cholesterinesterase und Cholesterin oxydierenden Enzymen, wie Cholesterin-Oxydase oder Cholesterin-Dehydrogenase, eine vollenzymatische Bestimmung auch von Cholesterinestern (DE-PS 22 64 847).

Als besonders geeignet hat sich im Rahmen der Cholesterinbestimmung mit Cholesterinesterase dabei das Enzym aus Mikroorganismen erwiesen (DE-OS 25 06 712.3).

Normalerweise erfolgt bei den bekannten Verfahren die Züchtung in einem Nährmedium, welches einen Induktor enthält (vgl. EP 0024345 B 1). Unter "Induktor" wird hierbei eine Substanz verstanden, welche den Mikroorganismus dazu anregt, das gewünschte Enzym überhaupt zu bilden oder in größerer Menge zu bilden als ohne Induktor. Denn normalerweise benötigen Mikroorganismen keine Cholesterinesterase, da ihnen genügend andere Nahrungsquellen zur Verfügung stehen und sie daher nicht auf die Verwertung von Cholesterinestern angewiesen sind. Induktoren bestehen daher meistens aus Cholesterinestern oder chemisch ähnlichen Verbindungen.

Es ist auch schon bekannt, daß bei Verwendung von bestimmten, Cholesterinestern chemisch fernstehenden, Induktoren ebenfalls gesteigerte Enzymaktivitäten erzielt werden können.

Verwendet man bekannte Induktoren auch als Kohlenstoffquelle, insbesondere als alleinige Kohlenstoffquelle, so sind die Ausbeuten an Cholesterin-Esterase jedoch verhältnismäßig niedrig.

Ein weiteres Problem der bekannten Verfahren liegt in der Aufarbeitung der fertig fermentierten Kulturbrühe.

Alle bekannten Induktoren sind weitgehend wasserunlöslich und werden nicht restlos verwertet. Dies macht es notwendig, durch Extraktion mit organischen Lösungsmitteln oder dergleichen die Induktor-Reste zu entfernen, was einen gravierenden negativen Einfluß auf die Stabilität des gereinigten Enzyms hat.

Der Erfindung liegt daher die Aufgabe zugrunde, die Nachteile der bekannten Verfahren zu beseitigen und ein Verfahren zu schaffen, welches mit einer einzigen Kohlenstoffquelle und ohne besondere Induktoren bessere Enzymausbeuten ermöglicht, als die bekannten Verfahren. Weiter hat die Erfindung zur Aufgabe, ein derartiges Verfahren zu schaffen, bei welchem die Aufarbeitung erleichtert ist und keine Extraktion mit organischen Lösungsmitteln erfordert.

Erfindungsgemäß wird diese Aufgabe gelöst durch ein Verfahren zur Gewinnung von Cholesterinesterase durch Züchtung eines geeigneten Mikroorganismus in einem geeigneten Nährmedium und Gewinnung des Enzyms aus der Kulturbrühe oder der Biomasse, welches dadurch gekennzeichnet ist, daß man die Züchtung in einem n-Alkane mit 10 bis 20 C-Atomen als einzige Kohlenstoffquelle enthaltenden Mineralsalzmedium als belüftete Submerskultur durchgeführt. Die Züchtung kann sowohl im batch-Verfahren wie auch im teil- oder vollkontinuierlichen Betrieb in einer oder mehreren Stufen durchgeführt werden. Geeignet sind hierfür sowohl ein chemostatischer wie auch ein turbidostatischer Betrieb.

Bei der Durchführung des erfindungsgemäßen Verfahrens werden die Mikroorganismen an das Kulturmedium, welches erfindungsgemäß als einzige Kohlenstoffquelle n-Alkane enthält, zuerst in üblicher Weise adaptiert, indem man sie in Kulturen zunehmend größeren Volumens anzüchtet. In der Regel wird zuerst auf festem Nähragar angezüchtet, dann zweckmäßig in Vollmedium in der Schüttelkultur vermehrt und dann auf das erfindungsgemäße n-Alkane enthaltende Mineralsalzmedium überimpft. In dieser Vorkultur züchtet man vorzugsweise so lange weiter, bis eine Cholesterin-Esterase-Aktivität von mindestens 5000 U/l erreicht ist.

In der Hauptkultur liegt die Temperatur zweckmäßig zwischen etwa 15 und 45 °C. Bevorzugt wird bei 25 bis 35 °C gearbeitet. Maximale Enzymausbeuten werden im allgemeinen unter diesen Bedingungen bei zwei- bis fünftägiger Kulturdauer erreicht, in vielen Fällen reichen zwei bis drei Tage Kultur aus. Die Cholesterinesterase tritt dabei sowohl im Medium, als auch in den Zellen auf. Bei zunehmender Züchtungsdauer verschiebt sich das Verhältnis der Enzymaktivität in der Biomasse zur Enzymaktivität in der Kulturbrühe zugunsten der letzteren, bis schließlich die gesamte Aktivität in der Kulturbrühe vorliegt. Gemäß einer bevorzugten Ausführungsform der Erfindung wird daher die Kultur so lange durchgeführt, bis praktisch die gesamte Aktivität in der Kulturbrühe vorliegt und aus dem Kulturfiltrat danach isoliert und gegebenenfalls gereinigt werden kann.

Der Übergang der Aktivität in die Kulturbrühe kann beispielsweise durch pH-Änderung, Änderung der Kulturtemperatur oder Zusatz von oberflächenaktiven Substanzen beschleunigt werden.

Die n-Alkankonzentration im Nährmedium wird in der Regel im Bereich zwischen 0,1 bis 5 % V/V gehalten. Diese Werte können jedoch auch unter- bzw. überschritten werden, was jedoch keine Vorteile ergibt.

Der pH-Wert wird zweckmäßig durch Zugabe eines geeigneten Korrekturmittels auf Werten zwischen etwa 5 und 9, vorzugsweise zwischen 6 und 8 gehalten.

Ein besonders bevorzugtes Nährmedium gemäß der Erfindung enthält abgesehen vom n-Alkan noch folgende Salze, bezogen auf 1000 Liter (1 m$^3$):

5 bis 10 kg, vorzugsweise 6 bis 8 kg $(NH_4)_2HPO_4$

1 bis 5 kg, vorzugsweise 2 bis 4 kg $KH_2PO_4$

0,2 bis 2, vorzugsweise 0,8 bis 1,2 kg $MgSO_4$

0,2 bis 2, vorzugsweise 0,8 bis 1,2 kg $CaCl_2$

0,01 bis 0,5, vorzugsweise 0,03 bis 0,15 kg NaCl

0,1 bis 1 l 1%ige $FeCl_3$-Lösung

0,1 bis 1 l 0,2%ige $CuCl_2$-Lösung

0,01 bis 1 l 1%ige $ZnSO_4$-Lösung sowie

Spuren der Elemente Mn, Co, Mo und B.

Als Mikroorganismen können im Prinzip solche verwendet werden, die einen die Aufarbeitung lohnenden Gehalt an Cholesterinesterase liefern. Eine größere Anzahl geeigneter Mikroorganismen ist bekannt, beispielsweise aus der DE-OS 25 06 712. Bevorzugt werden im Rahmen der Erfindung Pseudomonas spec. DSM 1280 und DSM 1281 verwendet.

Mit n-Alkan werden die besten Ergebnisse mit solchen Verbindungen oder Gemischen erzielt, die geradkettige n-Alkane mit 14 bis 18 C-Atomen, also insbesondere n-Tetradekan, n-Hexadekan und n-Octadekan enthalten.

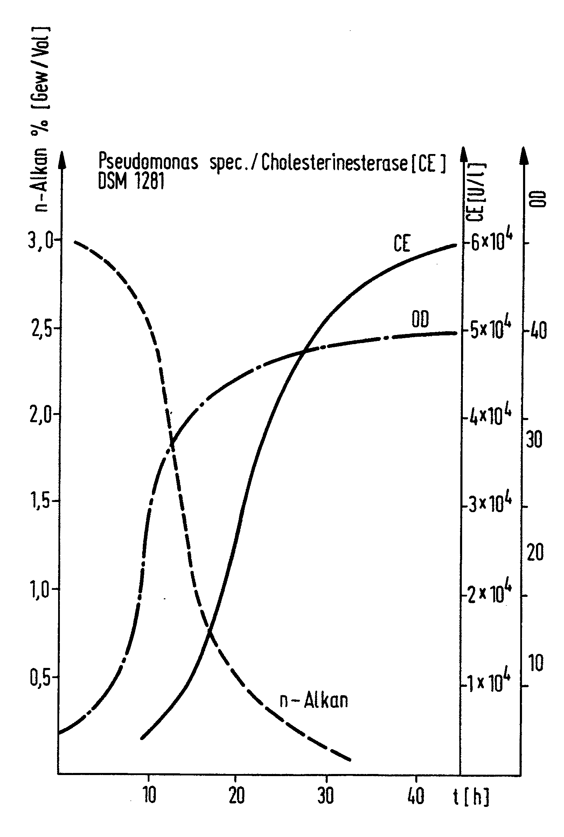

Das folgende Beispiel erläutert die Erfindung weiter in Verbindung mit der Zeichnung, in welcher die Abnahme an n-Alkan im Medium, die Zunahme von Trockenmasse g/l, Mikroorganismendichte (OD) und Enzymaktivität in der Kulturbrühe gegen die Zeit aufgetragen sind.

**Beispiel**

1. Nährboden

| Rohstoff | (kg/1 m³) Hauptfermentat |
|---|---|
| Di-Ammoniumhydrogenphosphat $(NH4)_2HPO_4$ | 7,0 |
| Kaliumhydrogenphosphat $KH_2PO_4$ | 3,0 |
| Magnesiumsulfat $MgSO_4 \times 7\ H_2O$ | 0,6 |
| Natriumchlorid NaCl | 0,05 |
| Eisen-III-Chlorid, $FeCl_3 x6H_2O$ (1%ig) | 0,1 Ltr. |
| Zinksulfat-7-hydrat, $ZnSO_4 x7H_2O$ (1%ig) | 0,1 " |
| Calciumchlorid $CaCl_2$ | 1,0 |
| n-Hexadecan (95%ig) olefinfrei | 30,0 |
| Wasserart: Trinkwasser | 1 m³ |
| pH (vor der Sterilisation mit $H_3PO_4$ einstellen) | 6,6 |

2. Anzucht und Vorkulturen

Schrägagarkulturen von Pseudomonas sp. DSM 1281 werden 72 Stunden bei 30°C bebrütet und anschließend bei 4°C aufbewahrt. Nährbodenzusammensetzung wie in 1) angegeben.

1. Flüssigführung:

100 ml Nährboden wie in 1) angegeben und 0,2 % (Gew./Vol.) Hefeextrakt in einem 300 ml-Schüttel-Kolben werden mit einer Öse von der Schrägagarkultur beimpft und bei 30°C, 130 UpM 72 Stunden inkubiert.

2. Flüssigführung:

4 x 1 1 Schüttel-Kolben mit je 250 ml Nährmedium werden von der 1. Flüssigführung im Verhältnis 1:100 beimpft, 72 Stunden bei 30°C und 130 UpM geschüttelt.
Der pH-Wert liegt dann bei 7,5 bis 8,0 und die Cholesterin-Esterase-Aktivität bei ca. 5000 U/Liter.
Diese Flüssigführung dient als Impfkultur für den 10 Liter-Fermenter.

3. Vorfermentation.

4

Verwendet wird ein 10 Liter-Fermenter, ausgestattet mit 4 Schikanen und 3 Scheibenrührern. Das Inoculum beträgt 1 %.

Die Laufzeit der Vorfermentation beträgt ca. 24 bis 48 Stunden. Die Cholesterin-Esterase-Aktivität liegt dann bei ca. $6 \times 10^4$ U/l.

4. Hauptfermentation

Verwendet wird ein 1 $m^3$-Fermenter, ausgestattet mit 3 Scheibenrührern, 4 Schikanen und einem mechanischen Schaumzerstörer.

Als Prozeßwasser wird Trinkwasser verwendet. Alle Nährboden-Komponenten werden im Fermenter gelöst und sterilisiert. Das n-Hexadecan wird separat mit ca. 10 % VE-Wasser sterilisiert und mit einer Temperatur größer 20°C dem Nährboden aseptisch zugesetzt. Die Fermentation wird 20 bis 50 Stunden bei 20 bis 37°C, 250 bis 400 UpM und 0,5 bis 1,5 VVM Luftzufuhr durchgeführt.

Der Fermentationsverlauf ist in der Zeichnung graphisch dargestellt. Die Ausbeute an Cholesterinesterase liegt bei $6,5 \times 10^4$ U/l.

**Ansprüche**

1. Verfahren zur Gewinnung von Cholesterinesterase durch Züchtung eines geeigneten Mikroorganismus in einem geeigneten Nährmedium und Gewinnung des Enzyms aus der Kulturbrühe oder der Biomasse, **dadurch gekennzeichnet,** daß man die Züchtung in einem n-Alkane mit 10 bis 20 C-Atomen als einzige Kohlenstoffquelle enthaltenden Mineralsalzmedium als belüftete Submerskultur durchführt.

2. Verfahren nach Anspruch 1, **dadurch gekennzeichnet,** daß man 2 bis 5 Tage bei 15 bis 45°C züchtet.

3. Verfahren nach Anspruch 2, **dadurch gekennzeichnet** daß man so lange züchtet, bis im wesentlichen die gesamte Enzymaktivität in der Kulturbrühe vorliegt.

4. Verfahren nach einem der vorhergehenden Ansprüche, **dadurch gekennzeichnet,** daß die n-Alkankonzentration im Nährmedium 0,1 bis 5 % V/V beträgt.

5. Verfahren nach einem der vorhergehenden Ansprüche, **dadurch gekennzeichnet,** daß man Mikroorganismen einsetzt, die in der Vorkultur auf dem n-Alkanmedium solange gezüchtet werden, bis eine Cholesterin-Esterase-Aktivität von mindestens 5000 U/l erreicht ist.

6. Verfahren nach einem der vorhergehenden Ansprüche, **dadurch gekennzeichnet,** daß man als n-Alkan ein solches mit 14 bis 18 C-Atomen oder ein Gemisch davon verwendet.

7. Verfahren nach einem der vorhergehenden Ansprüche, **dadurch gekennzeichnet,** daß man während der Züchtung einen pH von 5 bis 9 einhält.

8. Verfahren nach Anspruch 7, **dadurch gekennzeichnet,** daß man als Puffer Phosphatpuffer verwendet.

9. Verfahren nach einem der vorhergehenden Ansprüche, **dadurch gekennzeichnet,** daß man solche Salze verwendet, welche $NH_4$-, Fe-, Cu-, Zn-, Ca- und Mg-Ionen enthalten.

10. Verfahren nach einem der vorhergehenden Ansprüche, **dadurch gekennzeichnet,** daß man Pseudomonas sp. DSM 1280 oder 1281 züchtet.

**Claims**

1. Process for the obtaining of cholesterol esterase by culturing of a suitable micro-organism in a suitable nutrient medium and recovery of the enzyme from the culture broth or the biomass, characterised in

EP 0 188 770 B1

that the culturing is carried out in a mineral salt medium containing an n-alkane with 10 to 20 C-atoms as sole carbon source as aerated submersion culture.

2. Process according to claim 1, characterised in that one cultures for 2 to 5 days at 15 to 45° C.

3. Process according to claim 2, characterised in that one cultures until substantially the whole enzyme activity is present in the culture broth.

4. Process according to one of the preceding claims, characterised in that the n-alkane concentration in the nutrient medium amounts to 0.1 to 5% v/v.

5. Process according to one of the preceding claims, characterised in that micro-organisms are used which are cultured in the pre-culture on the n-alkane medium until a cholesterol esterase activity of at least 5000 U/l. is achieved.

6. Process according to one of the preceding claims, characterised in that, as n-alkane, one uses one with 14 to 18 C-atoms or a mixture thereof.

7. Process according to one of the preceding claims, characterised in that one maintains a pH of 5 to 9 during the culturing.

8. Process according to claim 7, characterised in that one uses phosphate buffer as buffer.

9. Process according to one of the preceding claims, characterised in that one uses those salts which contain $NH_4$, Fe, Cu, Zn, Ca and Mg ions.

10. Process according to one of the preceding claims, characterised in that one cultures Pseudomonas sp. DSM 1280 or 1281.

**Revendications**

1. Procédé pour la production de cholestérol estérase par culture d'un micro-organisme approprié dans un milieu nutritif approprié et extraction de l'enzyme à partir du bouillon de culture ou de la biomasse, caractérisé en ce qu'on effectue la culture sous forme de culture submergée, aérée, dans un milieu salin minéral contenant des n-alcanes avec 10 à 20 atomes de C comme source unique de carbone.

2. Procédé selon la revendication 1, caractérisé en ce qu'on cultive 2 à 5 jours à 15 jusqu'à 45° C.

3. Procédé selon la revendication 2, caractérisé en ce qu'on cultive jusqu'à ce qu'essentiellement toute l'activité enzymatique se trouve dans le bouillon de culture.

4. Procédé selon l'une des revendications précédentes, caractérisé en ce que la concentration en n-alcanes dans le milieu nutritif est de 0,1 à 5 % en volume/volume.

5. Procédé selon l'une des revendications précédentes, caractérisé en ce qu'on met en oeuvre des micro-organismes qui sont cultivés sur le milieu de n-alcanes jusqu'à ce qu'une activité en cholestérol estérase d'au moins 5000 U/l soit atteinte.

6. Procédé selon l'une des revendications précédentes, caractérisé en ce que l'on utilise comme n-alcane un n-alcane avec 14 jusqu'à 18 atomes de C ou un mélange de tels alcanes.

7. Procédé selon l'une des revendications précédentes, caractérisé en ce que pendant la culture, on maintient un pH de 5 à 9.

8. Procédé selon la revendication 7, caractérisé en ce qu'on utilise du tampon phosphate comme tampon.

9. Procédé selon l'une des revendications précédentes, caractérisé en ce que l'on utilise des sels qui

6

contiennent des ions $NH_4$, Fe, Cu, Zn, Ca et Mg.

**10.** Procédé selon l'une des revendications précédentes, caractérisé en ce que l'on cultive Pseudomonas sp. DSM 1280 ou 1281.